Europäisches Patentamt

European Patent Office

Office européen des brevets

(19)

(11) Publication number : **0 611 570 A1**

## (12) EUROPEAN PATENT APPLICATION

(21) Application number : **94810017.7**

(22) Date of filing : **14.01.94**

(51) Int. Cl.⁵ : **A61K 31/555**

(30) Priority : **19.01.93 GB 9300920**

(43) Date of publication of application :
**24.08.94 Bulletin 94/34**

(84) Designated Contracting States :
**AT BE CH DE DK ES FR GB GR IE IT LI LU NL PT SE**

(71) Applicant : **SANDOZ NUTRITION LTD.**
**Monbijoustrasse 118**
**CH-3001 Berne (CH)**

(72) Inventor : **Schneider, Heinz**
**Megglete 3**
**CH-1792 Cordast (CH)**

(74) Representative : **Smolders, Walter et al**
**Sandoz Technology LTD.,**
**Patents and Trademarks Division,**
**Lichtstrasse 35**
**CH-4002 Basle (CH)**

(54) Use of chromic tripicolinate in the treatment of obese diabetic patients.

(57) Use of chromic tripicolinate to maintain insulin sensitivity resulting from weight loss, to reduce body fat and to reduce coronary risk factors in obese diabetic patients.

EP 0 611 570 A1

The present invention relates to the use of chromic tripicolinate for weight reduction and weight maintenance of obese diabetic patients.

USP 5 087 624 discloses that chromic picolinate, preferably chromic tripicolinate, can be used as an anabolic agent in animals including humans to increase lean body mass and concomitantly decrease the percentage of body fat. This is supported by data presented in Example 5 and illustrated by Figures 12 and 13. Under the conditions of the study, the body weight of the group given chromic tripicolinate increased significantly by $2.18 \pm 1.34$ kg, the calculated lean body mass thereof increased significantly by 1.6 kg.

USP 5 087 624 also discloses a protocol (Example 4) comparing the effects of chromic tripicolinate on subjects with adult onset diabetes mellitus with that of placebo treated patients. The results show that no significant changes in weight, blood pressure, heart rate and temperature occur, but that both haemoglobin $A_1C$ and LDL decreased significantly and that HDL and cholesterol trends were favourable.

It is further suggested by USP 5 087 624 that chromic tripicolinate has Glucose Tolerance Factor activity and that it is more effective for reduction of elevated serum cholesterol, improvement of glucose tolerance and reduction in fasting hyperglycemia than other compounds of chromium. Chromic tripicolinate has accordingly been suggested for control of certain symptoms associated with maturity-onset diabetes.

Reduced insulin sensitivity or increased insulin resistance is associated with the occurrence of obesity, Type II diabetes, hyperlipidemia, hypertension and coronary artery disease.

Obese patients have an increased insulin concentration in their blood plasma, they are hyperinsulinemic.

Obese diabetic patients have an increased insulin and glucose in their blood plasma, they are both hyperinsulinemic and hyperglycemic.

It is well known that insulin-stimulated glucose uptake improves during weight loss of obese patients with non-insulin-dependent diabetes mellitus (NIDDM).

Weight loss is generally not sustained in these patients, and the beneficial effects of weight loss, such as decrease in fasting plasma glucose, lipids (cholesterol, triglycerides) and blood pressure, are only temporary.

It has now been found that the improved insulin sensitivity, achieved with weight loss of obese diabetic patients, can be maintained by administration of chromic tripicolinate throughout the weight maintenance phase (i.e. for the rest of patient's life).

This is i.a. illustrated by oral glucose tolerance tests (OGTT), carried out before, during and after weight loss. Such tests demonstrate a significantly lower incremental area under the blood glucose curve after weight loss with chromic tripicolinate compared to chromium yeast. Since the incremental areas under the corresponding plasma insulin curves are not significantly different for said two chromium sources, the tests indicate an improved insulin sensitivity of obese diabetics during treatment with chromic picolinate throughout the weight maintenance phase following weight loss.

The invention therefore provides a method of maintaining improved insulin sensitivity resulting from weight loss in obese diabetic patients, by administering during the weight maintenance phase of such patients an amount of chromic tripicolinate effective to maintain improved insulin sensitivity.

Improved insulin sensitivity on a continued basis is desirable to allow maintenance of the beneficial effects on pathophysiologies typical for obese diabetic patients, improved glucose tolerance in diabetics, lowered plasma lipids in hyperlipidemics, lowered blood pressure in hypertensives and reduced risk factors for developing coronary artery disease.

It has further been found that administration of chromic tripicolinate to obese diabetic patients subjected to a weight loss program results in a significantly increased reduction of body fat and thus in a significantly increased preservation of lean body mass.

Accordingly, the invention also provides an improved method of reducing body fat in obese diabetic patients under weight loss diet therapy by administering to such patients an amount of chromic tripicolinate effective to decrease the percent body fat.

For use in the methods of the invention, chromic tripicolinate may be administered enterally or parenterally; it is preferably administered enterally, more preferably orally. The chromic tripicolinate may be formulated in any suitable administration form, employing conventional formulation techniques and conventional formulation ingredients. It may for example be employed in nutritionally acceptable form by incorporation in a fibre supplement, a meal replacer, a drink mix or in pharmaceutically acceptable form, e.g. in tablet or capsule form in admixture with pharmaceutically acceptable carriers. The daily amount to be supplied for the above indicated methods of the invention will in general lie in the range of from about 50 to about 500 micrograms of chromium, e.g. 200 micrograms of chromium in the form of chromic tripicolinate.

The chromic picolinate may be administered in single dosage form or in the form of sub-units several times a day.

The following example illustrates the invention.

## EXAMPLE

### Study Design

Obese diabetic patients were evaluated and selected for a chromic tripicolinate study. After randomisation, 16 selected patients were divided into 2 groups of each 8 patients at the onset of the study in a double-blind study design.

Following a period of caloric restriction for 2 weeks, during intake of only 1000 kcal of regular food was allowed, the patients were given a liquid formula diet (MODIFAST[R], Sandoz Nutrition Ltd.) supplying 920 kcal/day for 2 weeks.

After this 4 weeks weight reduction phase, the patients were given a prescribed diet, supplying calories on an individual basis during a 6 weeks weight maintenance phase.

All patients received a fibre supplementation of 20 g/day as a carrier and 200 μm [Achromium/day during the 10 weeks study period. The control group (Group A of 8 patients) ingested the chromium in the form of chromium-yeast; the other group (Group B - of 8 patients) ingested the chromium in the form of chromic tripicolinate.

The body weight and body composition were determined at the beginning of the weight loss phase and subsequently every 2 weeks, i.e. at the end of study week 2, 4, 6, 8 and 10. The glucose tolerance of the patients was determined at the beginning and the end of the study.

### Results

### 1. Body weight/Body composition

The body composition was determined by establishing the percent body fat, the abdominal circumference (in cm) and the hip circumference (in cm). Percent body fat was derived from the sum of the triceps, subscapular and chest skin folds.

The results are shown in Tables A and B

## WEIGHT LOSS STUDY: CHROMIUM PICOLINATE (B) VS CHROMIUM YEAST (A)

### TABLE A

|  |  | % body fat (skin fold) | | |
| --- | --- | --- | --- | --- |
| Patient Group | Weight Loss (in kg) | Week 0 | Week 10 | Decrease |
| A(n=8) | 7.6+1.0 | 42.3+1.7 | 37.2+1.6 | 5.1+0.3 |
| B(n=8) | 7.5+0.9 | 42.3+1.3 | 35.4+1.5 | 7.0+0.7* |

* $p < 0.05$ (A vs B) unpaired t-Test (two tailed)

3

## <u>WEIGHT LOSS STUDY:   CHROMIUM PICOLINATE (B) VS CHROMIUM YEAST (A)</u>

### <u>TABLE B</u>

### Abdominal circumference (cm)

| Patient Group | Week 0 | Week 10 | Decrease | Decrease in Hip circumference (cm) |
|---|---|---|---|---|
| A(n=8) | 112.5+6.1 | 104.1+5.7 | 8.4+0.8 | 7.9+1.5 |
| B(n=8) | 114.8+5.3 | 102.4+4.3 | 12.4+1.8* | 8.4+1.1 |

**\* p = 0.066 (A vs B) unpaired t-Test (two tailed)**

Table A shows that, while the overall weight loss is essentially the same for both patient groups, the decrease in % body fat is significantly higher in patient group B, i.e. the group treated with chromic tripicolinate.

Table B shows that the reduction of the abdominal circumference was significantly greater with patients of group B than with patients of the control group (Group A). This is particularly important since abdominal fat deposits are associated with increased coronary risk factors and insulin resistance.

There was no significant difference between the decrease in hip circumference of patients of Group B and the control.

### 2. Glucose Tolerance

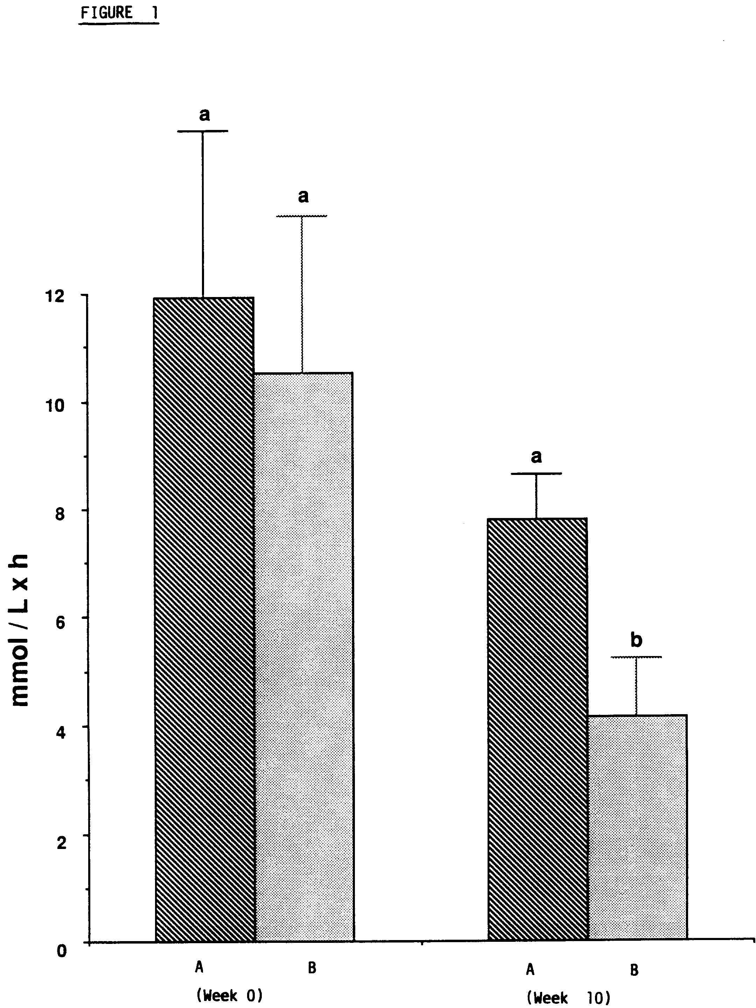

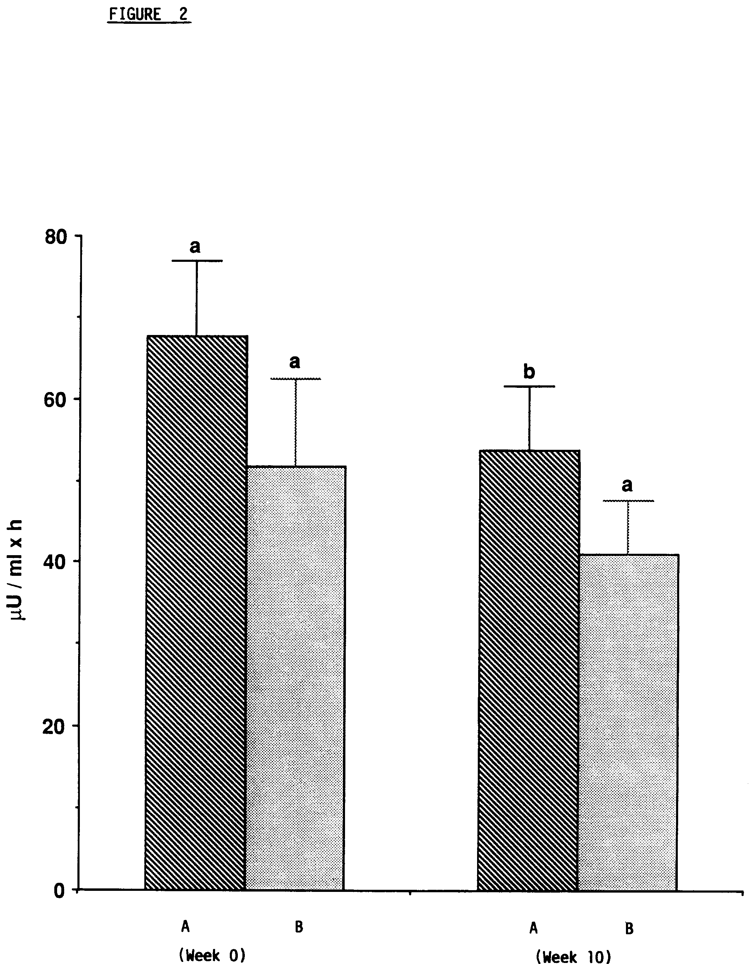

Glucose tolerance was established by determination of plasma glucose (in mmol/l blood plasma) and plasma insulin (in $\mu$U/ml of blood plasma) before and 30, 60, 90, 120 and 180 minutes after ingestion of 75 g glucose. The incremental areas under this established curves were then calculated. The results are shown in Fig. 1 and 2.

Fig. 1 shows the incremental areas under the plasma glucose curves before (week 0) and at the end of week 10 of the study. A shows the results obtained with chromium yeast (Group A), B the results obtained with chromic tripicolinate (Group B). According to ANOVA the incremental area under the blood glucose curve after weight loss with chromic tripicolinate is significantly smaller than with chromium yeast.

Fig. 2 shows the incremental areas under the plasma insulin curves before (week 0) and at the end of week 10 of the study. A shows the results obtained with chromium yeast (Group A), B the results with chromic tripicolinate (Group B). According to ANOVA there is no significant difference between the incremental area under the plasma insulin curve determined for Groups A and B.

### Claims

1. A method of maintaining improved insulin sensitivity resulting from weight loss in obese diabetic patients comprising administering during the weight maintenance phase of such patients an amount of chromic tripicolinate effective to maintain improved insulin sensitivity.

2. The method of Claim 1, wherein the chromic picolinate is administered orally.

4

3. The method of Claims 1 and 1, wherein from 50 to 500 micrograms chromium in the form of chromic picolinate are administered per day.

4. The use of chromic picolinate for the manufacture of a composition to maintain the improved level of insulin sensitivity in obese diabetic patients during the weight maintenance phase following the weight loss phase.

5. A method of reducing body fat in obese diabetic patients under weight loss therapy, comprising administering to such patients an amount of chromic tripicolinate effective to decrease the percent body fat.

6. The method of Claim 5, wherein the chromic picolinate is administered orally.

7. The method of Claims 5 and 6, wherein from 50 to 500 micrograms of chromium in the form of chromic picolinate are administered per day.

8. The use of chromic picolinate for the manufacture of a composition to decrease the percent body fat of obese diabetic patients under weight loss therapy.

9. The method of reducing coronary risk factors in obese diabetic patients, comprising administering to such patients an amount of chrome tripicolinate effective to reduce abdominal fat deposits.

FIGURE 1

FIGURE 2

EP 0 611 570 A1

**European Patent Office**

# PARTIAL EUROPEAN SEARCH REPORT

Application Number

which under Rule 45 of the European Patent Convention shall be considered, for the purposes of subsequent proceedings, as the European search report

EP 94 81 0017

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | J APPL NUTR,, VOL. 43, NO. 1, PAGE(S) 58-66, 1991 MCCARTY M F 'THE CASE FOR SUPPLEMENTATION CHROMIUM AND A SURVEY OF CLINICAL STUDIES WITH CHROMIUM PICOLINATE' * the whole document * --- | 1-9 | A61K31/555 |
| Y | US-A-5 087 623 (H. BOYNTON ET AL.) 11 February 1992 * the whole document * --- | 1-9 | |
| D,Y | US-A-5 087 624 (H. BOYNTON ET AL.) 11 February 1992 * the whole document * --- | 1-9 | |
| Y | J INORG BIOCHEM (UNITED STATES), JUN 1992, VOL. 46, NO. 4, PAGE(S) 243-50, Evans GW et al 'Chromium picolinate increases membrane fluidity and rate of insulin internalization' * the whole document * --- | 1,4 | |
| | -/-- | | **TECHNICAL FIELDS SEARCHED (Int.Cl.5)** A61K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 26 April 1994 | Krautbauer, B |

EPO FORM 1503 03.82 (P04C07)

European Patent Office

**PARTIAL EUROPEAN SEARCH REPORT**

Application Number

EP 94 81 0017

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | MEETING OF THE FEDERATION OF AMERICAN SOCIETIES FOR EXPERIMENTAL BIOLOGY (FASEB) PART II, ANAHEIM, CALIFORNIA, USA, APRIL,, VOL. 6, NO. 5, PAGE(S) A1665, 1992 EVANS G W et al 'COMPOSITION AND BIOLOGICAL ACTIVITY OF CHROMIUM PYRIDINE CARBOXYLATE COMPLEXES' * abstract no. 4220 * | 1,4,5,8 | |
| P,Y | J. OPTIM. NUTR., 1993, VOL. 2, NO. 1, PAGE(S) 36-53 McCarty, Mark F. 'Hypothesis: sensitization of insulin-dependent hypothalamic glucoreceptors may account for the fat-reducing effects of chromium picolinate' * the whole document * | 1-9 | |
| P,Y | MED HYPOTHESES (ENGLAND), OCT 1993, VOL. 41, NO. 4, PAGE(S) 308-15, McCarty MF 'Insulin resistance in Mexican Americans--a precursor to obesity and diabetes?' * the whole document * | 1-9 | TECHNICAL FIELDS SEARCHED (Int.Cl.5) |
| Y | WEST. J. MED. (USA), 1991, VOL. 155, NO. 5, PAGE(S) 549, Evans G.W. 'An inexpensive, convenient adjunct for the treatment of diabetes (4)' * the whole document * | 1-4 | |
| Y | CURR THER RES,, VOL. 51, NO. 2, PAGE(S) 261-274, 1992 KAATS G R et al 'THE SHORT-TERM THERAPEUTIC EFFICACY OF TREATING OBESITY WITH A PLAN OF IMPROVED NUTRITION AND MODERATE CALORIC RESTRICTION' * the whole document * | 1-9 | |

EPO FORM 1503 03.82 (P04C10)

9

EP 94 81 0017

-C-


Remark: Although claims 1-3,5-7,9
        are directed to a method of
        treatment of the human/animal
        body (Art. 52(4) EPC) the search
        has been carried out and based on
        the alleged effects of the
        compound/composition